# EUROPEAN PATENT APPLICATION

(11) **EP 4 435 011 A1**
(43) Date of publication of application: **25.09.2024**
(21) Application number: 22892171.4
(22) Date of filing: 15.11.2022
(51) Int. Cl.: C07K 19/00, C07K 16/28, C07K 14/54, C07K 14/715, C12N 15/62, C12N 15/85, A61K 38/20, A61K 39/395, A61P 35/00

(54) **FUSION PROTEIN CONSTRUCT TAKING INTERLEUKIN 15 AS ACTIVE INGREDIENT AND USE THEREOF**

(30) Priority: 15.11.2021 CN 202111346279; 23.08.2022 CN 202211013404
(71) Applicant: INSTITUTE OF BIOPHYSICS, CHINESE ACADEMY OF SCIENCES, Chaoyang District Beijing 100101 (CN)
(72) Inventor: FU, Yangxin, Beijing 100101 (CN); PENG, Hua, Beijing 100101 (CN); SHEN, Jiao, Beijing 100101 (CN)
(74) Representative: J A Kemp LLP
(86) International application number: PCT/CN2022/132019
(87) International publication number: WO 2023/083379

(57) **Abstract**

The present invention relates to a fusion protein construct taking interleukin 15 as an active ingredient and the use thereof. Specifically, the fusion protein construct comprises: (1) a first structural unit: an α subunit sushi domain of an interleukin 15 (IL-15) receptor; (2) a second structural unit: IL-15; and (3) a third structural unit: an antibody Fc or a mutated Fc fragment; and a key linking fragment 1, wherein the amino acid sequence of the linking fragment 1 is an integer multiple repetition of GGGGS.

## Description

### TECHNICAL FIELD

The invention belongs to the field of medicine and biology, and specifically relates to a fusion protein construct with interleukin-15 as an active ingredient and use thereof.

### BACKGROUND

With the rapid development of immune checkpoint therapy, PD-1/PD-L1 blocking antibody has become one of the important methods in clinical treatment. The PD-1/PD-L1 immune-blocking antibody relieves the inhibition of tumor on tumor infiltrating lymphocytes (TILs), and resumes the function of lymphocytes to recognize and kill tumors. However, in the later stages of treatment, due to excessive activation, T cells gradually enter a state of exhaustion and lose their function. The patients with tumors in clinical trials generally have a low response rate to PD-1/PD-L1 therapy, and are at risk of resistance or recurrence. Therefore, how to eliminate the bottleneck in the clinical application of PD-1/PD-L1 has become the most important issue in the current immunotherapy.

T cell growth factor has become an important target of immunotherapy because of its ability to expand T lymphocytes. Among them, IL-2 is a cytokine required for the expansion of T cells and one of the earliest immunotherapeutic drugs approved by the U.S. Food and Drug Administration (FDA) for malignant melanoma and kidney cancer. However, IL-2 has not been widely used in clinic for the following reasons: (1) short half-life; (2) activation of immunosuppressive regulatory T cells (Tregs); (3) elimination of activated effector T cells by activation-induced cell death (AICD); (4) severe toxic side effects caused by activation of vascular endothelial cells. IL-15 is another important T cell growth factor and a pleiotropic cytokine, which can maintain and activate innate and adaptive immune responses. IL-15 can promote the activation, proliferation and survival of CD8+ T cells; and activate and maintain memory T cells. IL-15 can also promote the activation and proliferation of NK and NKT cells. In addition, IL-15 can also promote the activation, proliferation and differentiation of dendritic cells in an autocrine manner, promote the expression of MHC-II and CD80/CD86, and improve the antigen cross-presentation of dendritic cells. Compared with IL-2, IL-15 has the following advantages in tumor therapy: (1) IL-15 can avoid activation-induced cell death, and induce the generation and steady-state proliferation of long-lived memory T cells. (2) IL-15 does not expand Tregs. (3) IL-2 acts on vascular endothelial cells thereby causing systemic toxicity, while IL-15 does not cause this side effect. Therefore, IL-15 is currently a potential cytokine for tumor immunotherapy.

The clinical use of IL-15 is mainly limited by its short half-life and low therapeutic effect. Some researchers linked IL-15 mutants to IL-15Rα and IgG1 Fc domain (ALT-803) to extend the half-life. However, compared with immune checkpoint blockade therapy, the combination with ALT-803 does not bring great advantages. At the same time, an increased number of systemic circulating NK and CD8+ T cells was detected in all patients, indicating the significant toxic side effects of IL-15 at the therapeutic dose. At present, many preclinical studies used IL-15 mutation to reduce the binding of IL-15 to its receptor. Although it can reduce the binding of IL-15 to peripheral NK cells, it will also affect the anti-tumor activity of IL-15. Moreover, artificially modified IL-15 is also more likely to induce anti-IL-15 antibodies in the human body, thereby further compromising the therapeutic effect of IL-15.

As a popular target, PD-1 *per se* has great advantages. PD-1 has a higher expression in tumors compared with other tissues throughout the body. More importantly, CD8+ T cells in the tumor express high levels of PD-1, which enables αPD-1 antibodies to directly act on the killer T cell population. At the same time, αPD-1 blocking antibodies can also block the binding of PD-1 and PD-L1, relieve the inhibition of T cells, and further enhance the anti-tumor ability of T cells.

As mentioned above, the effect of PD-1/PD-L1 antibody blocking therapy is not significant, probably due to the gradual T cell exhaustion. As a T cell agonist, IL-15 can promote the proliferation of CD8+ T cells, which may enhance the killing ability of T cells during PD-1/PD-L1 antibody blocking therapy. The concept of PD-1/PD-L1 antibody-cytokine fusion proteins is very common, but the most forms of fusion proteins still produce significant toxic side effects. The reason for this is the wide distribution of cytokine receptors (such as IL-15 receptors, which are expressed on T cells and NK cells) and the high affinity of cytokines to their receptors (Kd ~ 10⁻⁹M). As a result, in the antibody-cytokine fusion proteins, the affinity between the cytokine and the receptor is likely to be greater than the affinity between the antibody and the antigen, which cannot produce an effective tumor-targeting effect, thereby bringing significant toxic side effects during systemic injection.

The advantage of the fusion proteins constructed in the present disclosure is that the length of the first linker between the hIgG globulin and IL-15 is adjusted to hinder the binding of IL-15 to its receptor IL-2/15Rβ (CD122) by steric hindrance effect. When circulating peripherally, the fusion protein targeting drug reduces the binding to IL-15 receptor-expressing NK cells, thereby reducing peripheral toxic side effects. In the tumor microenvironment, the fusion protein specifically binds to CD8+ T cells highly expressing PD-1 through the αPD-1 antibody, and at the same time assists the binding of IL-15 to the receptor on CD8+T cells by utilizing the high affinity of the αPD-1 antibody, thus specifically activating the CD8+T cells. While utilizing the αPD-1 antibody to block the inhibition of PD-1/PD-L1 on T cells, the fusion protein further promotes the proliferation of T cells and enhances the killing ability of T cells through the signaling of IL-15, which enhances the anti-tumor effect of the fusion protein targeting drug while eliminating the peripheral toxic side effects.

### SUMMARY

The present disclosure firstly relates to a fusion protein, comprising blocks of:
(1) a first structural unit: a sushi domain of an interleukin 15 (IL-15) receptor subunit α;
(2) a second structural unit: IL-15;
(3) a third structural unit located at N-terminus of the fusion protein: an Fc fragment or a mutated Fc fragment of an antibody; and
(4) a second linker for linking the first and second structural units;

a first linker links the second and third structural units where C-terminus of the fusion protein is the first structural unit;
the first linker links the first and third structural units where the C-terminus of the fusion protein is the second structural unit.

The first linker has an amino acid sequence which is an integer multiple repeat of GGGGS, represented by (G₄S)ₙ; preferably, n is any integer from 1 to 7; more preferably, n is any integer from 1 to 5; most preferably, n is 3.

Further, the fusion protein may further comprise blocks of:
(5) a fourth structural unit linked to the N-terminus of the fusion protein (the N-terminus of the third structural unit): a Fab of a therapeutic antibody;
the therapeutic antibody includes but is not limited to: anti-PD1/PD-L1 antibody, Her2 antibody, anti-CD20 antibody, anti-CD19 antibody, anti-RANKL antibody, anti-VEGFR antibody, and anti-EGFR antibody;
preferably, the Fab of the therapeutic antibody is an anti-PD-1 Fab (a Fab of a PD1 antibody).

The anti-PD-1 Fab comprises a heavy chain (variable region + constant region) and a light chain (variable region + constant region), wherein the heavy chain is located at the N-terminus of the fusion protein;
preferably, the anti-PD-1 Fab is a murine- or human-derived anti-PD-1 Fab;
more preferably, the murine- or human-derived anti-PD-1 Fab has a light chain with an amino acid sequence as shown in Seq ID No.4, Seq ID No.18, or Seq ID No.19; the anti-PD-1 Fab has a heavy chain with an amino acid sequence as shown in Seq ID No.5, Seq ID No.20, or Seq ID No.21.
   Seq ID No.4: 
YELTQPPSASVNVGETVKITCSGDQLPKYFADWFHQRSDQTILQVIYDDNKRPSGIPERISGSSSGTTA
TLTIRDVRAEDEGDYYCFSGYVDSDSKLYVFGSGTQLTVLGRTVAAPSVFIFPPSDEQLKSGTASVVCLLN
NFYPREAKVQWKVDNALQSGNSQESVTEQDSKDSTYSLSSTLTLSKADYEKHKVYACEVTHQGLSSPVT
KSFNRGEC
   Seq ID No.18: 
EIVLTQSPATLSLSPGERATLSCRASKGVSTSGYSYLHWYQQKPGQAPRLLIYLASYLESGVPARFSGS
GSGTDFTLTISSLEPEDFAVYYCQHSRDLPLTFGGGTKVEIKRTVAAPSVFIFPPSDEQLKSGTASVVCLLNNF
YPREAKVQWKVDNALQSGNSQESVTEQDSKDSTYSLSSTLTLSKADYEKHKVYACEVTHQGLSSPVTKS
FNRGEC
   Seq ID No.19: 
EIVLTQSPATLSLSPGERATLSCRASQSVSSYLAWYQQKPGQAPRLLIYDASNRATGIPARFSGSGSGT
DFTLTISSLEPEDFAVYYCQQSSNWPRTFGQGTKVEIKRTVAAPSVFIFPPSDEQLKSGTASVVCLLNNFYPR
 
EAKVQWKVDNALQSGNSQESVTEQDSKDSTYSLSSTLTLSKADYEKHKVYACEVTHQGLSSPVTKSFNR
GEC
   Seq ID No.5: 
EVRLLESGGGLVKPEGSLKLSCVASGFTFSDYFMSWVRQAPGKGLEWVAHIYTKSYNYATYYSGSV
KGRFTISRDDSRSMVYLQMNNLRTEDTATYYCTRDGSGYPSLDFWGQGTQVTVSSAASTKGPSVFPLAPS
SKSTSGGTAALGCLVKDYFPEPVTVSWNSGALTSGVHTFPAVLQSSGLYSLSSVVTVPSSSLGTQTYICNV
NHKPSNTKVDKRVEPKSC
   Seq ID NO.20: 
QVQLVQSGVEVKKPGASVKVSCKASGYTFTNYYMYWVRQAPGQGLEWMGGINPSNGGTNFNEKF
KNRVTLTTDSSTTTAYMELKSLQFDDTAVYYCARRDYRFDMGFDYWGQGTTVTVSSASTKGPSVFPLAP
CSRSTSESTAALGCLVKDYFPEPVTVSWNSGALTSGVHTFPAVLQSSGLYSLSSVVTVPSSSLGTKTYTCNV
DHKPSNTKVDKRV
   Seq ID No.21:

QVQLVESGGGVVQPGRSLRLDCKASGITFSNSGMHWVRQAPGKGLEWVAVIWYDGSKRYYADSVK
GRFTISRDNSKNTLFLQMNSLRAEDTAVYYCATNDDYWGQGTLVTVSSASTKGPSVFPLAPCSRSTSESTA
ALGCLVKDYFPEPVTVSWNSGALTSGVHTFPAVLQSSGLYSLSSVVTVPSSSLGTKTYTCNVDHKPSNTK
VDKRVESK

The IL-15 is a murine- or human-derived IL-15, having an amino acid sequence as shown in Seq ID No.1 or Seq ID No.15;
Seq ID No.1: 
NWIDVRYDLEKIESLIQSIHIDTTLYTDSDFHPSCKVTAMNCFLLELQVILHEYSNMTLNETVRNVLYL
ANSTLSSNKNVAESGCKECEELEEKTFTEFLQSFIRIVQMFINTS
Seq ID No.15:

NWVNVISDLKKIEDLIQSMHIDATLYTESDVHPSCKVTAMKCFLLELQVISLESGDASIHDTVENLIIL
ANNSLSSNGNVTESGCKECEELEEKNIKEFLQSFVHIVQMFINTS

The sushi domain of the murine- or human-derived IL-15 receptor subunit α has an amino acid sequence as shown in Seq ID No.3 or Seq ID No.17;
Seq ID No.3: 
GTTCPPPVSIEHADIRVKNYSVNSRERYVCNSGFKRKAGTSTLIECVINKNTNVAHWTTPSLKCIRDPS
LAHYSPVPT
Seq ID No.17:

ITCPPPMSVEHADIWVKSYSLYSRERYICNSGFKRKAGTSSLTECVLNKATNVAHWTTPSLKCIRDPAL
VHQRPAPP

The Fc fragment or the mutated Fc fragment has an amino acid sequence as shown in Seq ID No.2 or Seq ID No.16;
Seq ID No.2: 
EPKSSDKTHTCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKAKGQPREPQVYTL
PPSRDELTKNQVSLTCLVKGFYPSDIAVEWESNGQPENNYKTTPPVLDSDGSFFLYSKLTVDKSRWQQGN
VFSCSVMHEALHNHYTQK
Seq ID No.16:

EPKSSDKTHTCPPCPAPEAAGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALGAPIEKTISKAKGQPREPQVYTL
PPSRDELTKNQVSLTCLVKGFYPSDIAVEWESNGQPENNYKTTPPVLDSDGSFFLYSKLTVDKSRWQQGN
VFSCSVMHEALHNHYTQK

The second linker has an amino acid sequence as shown in Seq ID No.6.
Seq ID No.6:
SGGGSGGGGSGGGGSGGGGSGGGSLQ

The present disclosure also relates to a homodimer composed of the fusion protein, preferably, the homodimer's monomers are linked to each other through dimerization of the third structural units.

More preferably, the homodimer is:
(1) homodimer 1 (Fc-G₄S-Rα-IL-15):
   the fusion protein as the monomer, from N-terminus to C-terminus, comprises: a human-derived IgG1-Fc, a first linker (G₄S), a sushi domain of an IL-15 receptor subunit α, a second linker and IL-15; preferably, homodimer 1 has an amino acid sequence as shown in SEQ ID No.7; 
SEQ ID No.7:
KLATMETDTLLLWVLLLWVPGSTGEPKSSDKTHTCPPCPAPELLGGPSVFLFPPKPKDTLMISRTP
EVTCVVVDVSHEDPEVKFNWYVDGVEVHNAKTKPREEQYNSTYRVVSVLTVLHQDWLNGKEYKCKVS
NKALPAPIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKGGGGSGTTCPPPVSIEHADIRVK
NYSVNSRERYVCNSGFKRKAGTSTLIECVINKNTNVAHWTTPSLKCIRDPSLAHYSPVPTSGGGSGGG
GSGGGGSGGGGSGGGSLQNWIDVRYDLEKIESLIQSIHIDTTLYTDSDFHPSCKVTAMNCFLLELQVILH
EYSNMTLNETVRNVLYLANSTLSSNKNVAESGCKECEELEEKTFTEFLQSFIRIVQMFINTS
(2) homodimer 2 (Fc-G₄S-IL-15-Rα):
   the fusion protein as the monomer, from N-terminus to C-terminus, comprises: a human-derived IgG1-Fc, a first linker (G₄S), IL-15, a second linker, and a sushi domain of an IL-15 receptor subunit α; preferably, homodimer 2 has an amino acid sequence structure as shown in SEQ ID No.8; 
SEQ ID No.8:
KLATMETDTLLLWVLLLWVPGSTGEPKSSDKTHTCPPCPAPELLGGPSVFLFPPKPKDTLMISRTP
EVTCVVVDVSHEDPEVKFNWYVDGVEVHNAKTKPREEQYNSTYRVVSVLTVLHQDWLNGKEYKCKVS
NKALPAPIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKGGGGSNWIDVRYDLEKIESLIQS
IHIDTTLYTDSDFHPSCKVTAMNCFLLELQVILHEYSNMTLNETVRNVLYLANSTLSSNKNVAESGCKEC
EELEEKTFTEFLQSFIRIVQMFINTSSGGGSGGGSGGGGSGGGGSGGGSLQGTTCPPPVSIEHADIR
VKNYSVNSRERYVCNSGFKRKAGTSTLIECVINKNTNVAHWTTPSLKCIRDPSLAHYSPVPT
(3) homodimer 3 (anti-PD-1 Fab Fc-G₄S-Rα-IL-15):
   the fusion protein as the monomer, from N-terminus to C-terminus, comprises: an anti-PD-1 Fab, a human-derived IgG1-Fc, a first linker (G₄S), a sushi domain of an IL-15 receptor subunit α, a second linker, and IL-15; preferably, homodimer 3 has an amino acid sequence as shown in SEQ ID No.9; 
SEQ ID No.9:
YELTQPPSASVNVGETVKITCSGDQLPKYFADWFHQRSDQTILQVIYDDNKRPSGIPERISGSSSG
TTATLTIRDVRAEDEGDYYCFSGYVDSDSKLYVFGSGTQLTVLGRTVAAPSVFIFPPSDEQLKSGTASVV
CLLNNFYPREAKVQWKVDNALQSGNSQESVTEQDSKDSTYSLSSTLTLSKADYEKHKVYACEVTHQGL
SSPVTKSFNRGECKLATMETDTLLLWVLLLWVPGSTGEVRLLESGGGLVKPEGSLKLSCVASGFTFSDY
FMSWVRQAPGKGLEWVAHIYTKSYNYATYYSGSVKGRFTISRDDSRSMVYLQMNNLRTEDTATYYCTR
DGSGYPSLDFWGQGTQVTVSSAASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKRVEPKSCRTEPKSSDKTH
TCPPCPAPELLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTLPPSRDELTKN
QVSLTCLVKGFYPSDIAVEWESNGQPENNYKTTPPVLDSDGSFFLYSKLTVDKSRWQQGNVFSCSVMH
EALHNHYTQKGGGGSGTTCPPPVSIEHADIRVKNYSVNSRERYVCNSGFKRKAGTSTLIECVINKNTNV
AHWTTPSLKCIRDPSLAHYSPVPTSGGGSGGGGSGGGGSGGGGSGGGSLQNWIDVRYDLEKIESLIQ
SIHIDTTLYTDSDFHPSCKVTAMNCFLLELQVILHEYSNMTLNETVRNVLYLANSTLSSNKNVAESGCKE
CEELEEKTFTEFLQSFIRIVQMFINTS
(4) homodimer 4 (anti-PD-1 Fab Fc-G₄S-IL-15-Rα):
   the fusion protein as the monomer, from N-terminus to C-terminus, comprises: an anti-PD-1 Fab, a human-derived IgG1-Fc, a first linker (G₄S), IL-15, a second linker, and a sushi domain of an IL-15 receptor subunit α, preferably, homodimer 4 has an amino acid sequence as shown in SEQ ID No.10; 
SEQ ID No.10:
YELTQPPSASVNVGETVKITCSGDQLPKYFADWFHQRSDQTILQVIYDDNKRPSGIPERISGSSSG
TTATLTIRDVRAEDEGDYYCFSGYVDSDSKLYVFGSGTQLTVLGRTVAAPSVFIFPPSDEQLKSGTASVV
CLLNNFYPREAKVQWKVDNALQSGNSQESVTEQDSKDSTYSLSSTLTLSKADYEKHKVYACEVTHQGL
SSPVTKSFNRGECKLATMETDTLLLWVLLLWVPGSTGEVRLLESGGGLVKPEGSLKLSCVASGFTFSDY
FMSWVRQAPGKGLEWVAHIYTKSYNYATYYSGSVKGRFTISRDDSRSMVYLQMNNLRTEDTATYYCTR
DGSGYPSLDFWGQGTQVTVSSAASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKRVEPKSCRTEPKSSDKTH
TCPPCPAPELLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTLPPSRDELTKN
QVSLTCLVKGFYPSDIAVEWESNGQPENNYKTTPPVLDSDGSFFLYSKLTVDKSRWQQGNVFSCSVMH
EALHNHYTQKGGGGSNWIDVRYDLEKIESLIQSIHIDTTLYTDSDFHPSCKVTAMNCFLLELQVILHEYSN
MTLNETVRNVLYLANSTLSSNKNVAESGCKECEELEEKTFTEFLQSFIRIVQMFINTSSGGGSGGGGSG
GGGSGGGGSGGGSLQGTTCPPPVSIEHADIRVKNYSVNSRERYVCNSGFKRKAGTSTLIECVINKNTN
VAHWTTPSLKCIRDPSLAHYSPVPT
(5) homodimer 5 (Fc-(G₄S)₃-IL-15-Rα):
   the monomer, from N-terminus to C-terminus, comprises: a human-derived IgG1-Fc, a first linker ((G₄S)₃), a murine- or human-derived IL-15, a second linker, and a sushi domain of an IL-15 receptor subunit α; preferably, homodimer 5 has an amino acid sequence as shown in SEQ ID No.11; 
SEQ ID No.11:
KLATMETDTLLLWVLLLWVPGSTGEPKSSDKTHTCPPCPAPELLGGPSVFLFPPKPKDTLMISRTP
EVTCVVVDVSHEDPEVKFNWYVDGVEVHNAKTKPREEQYNSTYRVVSVLTVLHQDWLNGKEYKCKVS
NKALPAPIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKGGGGSGGGGSGGGGSNWIDV
RYDLEKIESLIQSIHIDTTLYTDSDFHPSCKVTAMNCFLLELQVILHEYSNMTLNETVRNVLYLANSTLSSN
KNVAESGCKECEELEEKTFTEFLQSFIRIVQMFINTSSGGGSGGGGSGGGGSGGGGSGGGSLQGTTC
PPPVSIEHADIRVKNYSVNSRERYVCNSGFKRKAGTSTLIECVINKNTNVAHWTTPSLKCIRDPSLAHYS
PVPT
(6) homodimer 6 (anti-PD-1 Fab Fc-(G₄S)₃-IL-15-Rα):
   the monomer, from N-terminus to C-terminus, comprises: an anti-PD-1 Fab, a human-derived IgG1-Fc, a first linker ((G₄S)₃), IL-15, a second linker, and a sushi domain of an IL-15 receptor subunit α; preferably, homodimer 6 has an amino acid sequence as shown in SEQ ID No.12; 
SEQ ID No.12:
YELTQPPSASVNVGETVKITCSGDQLPKYFADWFHQRSDQTILQVIYDDNKRPSGIPERISGSSSG
TTATLTIRDVRAEDEGDYYCFSGYVDSDSKLYVFGSGTQLTVLGRTVAAPSVFIFPPSDEQLKSGTASVV
CLLNNFYPREAKVQWKVDNALQSGNSQESVTEQDSKDSTYSLSSTLTLSKADYEKHKVYACEVTHQGL
SSPVTKSFNRGECKLATMETDTLLLWVLLLWVPGSTGEVRLLESGGGLVKPEGSLKLSCVASGFTFSDY
FMSWVRQAPGKGLEWVAHIYTKSYNYATYYSGSVKGRFTISRDDSRSMVYLQMNNLRTEDTATYYCTR
DGSGYPSLDFWGQGTQVTVSSAASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKRVEPKSCRTEPKSSDKTH
TCPPCPAPELLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTLPPSRDELTKN
QVSLTCLVKGFYPSDIAVEWESNGQPENNYKTTPPVLDSDGSFFLYSKLTVDKSRWQQGNVFSCSVMH
EALHNHYTQKGGGGSGGGGSGGGGSNWVDVRYDLEKIESLIQSIHIDTTLYTDSDFHPSCKVTAMNCFL
LELQVILHEYSNMTLNETVRNVLYLANSTLSSNKNVAESGCKECEELEEKTFTEFLQSFIRIVQMFINTSS
GGGSGGGGSGGGGSGGGGSGGGSLQGTTCPPPVSIEHADIRVKNYSVNSRERYVCNSGFKRKAGT
STLIECVINKNTNVAHWTTPSLKCIRDPSLAHYSPVPT
(7) homodimer 7 (Fc-(G₄S)₅-IL-15-Rα):
   the monomer, from N-terminus to C-terminus, comprises: a human-derived IgG1-Fc, a first linker ((G₄S)₅), IL-15, a second linker, and a sushi domain of an IL-15 receptor subunit α; preferably, homodimer 7 has an amino acid sequence as shown in SEQ ID No.13; 
SEQ ID No.13:
KLATMETDTLLLWVLLLWVPGSTGEPKSSDKTHTCPPCPAPELLGGPSVFLFPPKPKDTLMISRTP
EVTCVVVDVSHEDPEVKFNWYVDGVEVHNAKTKPREEQYNSTYRVVSVLTVLHQDWLNGKEYKCKVS
NKALPAPIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKGGGGSGGGGSGGGGSGGGGS
GGGGSNWIDVRYDLEKIESLIQSIHIDTTLYTDSDFHPSCKVTAMNCFLLELQVILHEYSNMTLNETVRNV
LYLANSTLSSNKNVAESGCKECEELEEKTFTEFLQSFIRIVQMFINTSSGGGSGGGGSGGGGSGGGGS
GGGSLQGTTCPPPVSIEHADIRVKNYSVNSRERYVCNSGFKRKAGTSTLIECVINKNTNVAHWTTPSLK
CIRDPSLAHYSPVPT
(8) homodimer 8 (anti-PD-1 Fab Fc-(G₄S)₅-IL-15-Rα):
   the monomer, from N-terminus to C-terminus, comprises: an anti-PD-1 Fab, a human-derived IgG1-Fc, a first linker ((G₄S)₅), IL-15, a second linker, and a sushi domain of an IL-15 receptor subunit α; preferably, homodimer 8 has an amino acid sequence as shown in SEQ ID No.14.

SEQ ID No.14:
YELTQPPSASVNVGETVKITCSGDQLPKYFADWFHQRSDQTILQVIYDDNKRPSGIPERISGSSSGTTA
TLTIRDVRAEDEGDYYCFSGYVDSDSKLYVFGSGTQLTVLGRTVAAPSVFIFPPSDEQLKSGTASVVCLLN
NFYPREAKVQWKVDNALQSGNSQESVTEQDSKDSTYSLSSTLTLSKADYEKHKVYACEVTHQGLSSPVT
KSFNRGECKLATMETDTLLLWVLLLWVPGSTGEVRLLESGGGLVKPEGSLKLSCVASGFTFSDYFMSWV
RQAPGKGLEWVAHIYTKSYNYATYYSGSVKGRFTISRDDSRSMVYLQMNNLRTEDTATYYCTRDGSGYP
SLDFWGQGTQVTVSSAASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCRTEPKSSDKTHTCPPCPAPELLG
GPSVFLFPPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSRDELTKNQVSLTCLVKGFYPS
DIAVEWESNGQPENNYKTTPPVLDSDGSFFLYSKLTVDKSRWQQGNVFSCSVMHEALHNHYTQKGGGG
SGGGGSGGGGSGGGGSGGGGSNWIDVRYDLEKIESLIQSIHIDTTLYTDSDFHPSCKVTAMNCFLLELQVI
LHEYSNMTLNETVRNVLYLANSTLSSNKNVAESGCKECEELEEKTFTEFLQSFIRIVQMFINTSSGGGSGG
GGSGGGGSGGGGSGGGSLQGTTCPPPVSIEHADIRVKNYSVNSRERYVCNSGFKRKAGTSTLIECVINKN
TNVAHWTTPSLKCIRDPSLAHYSPVPT

The present disclosure also relates to a nucleotide fragment encoding the fusion protein.

The present disclosure also relates to use of the fusion protein and the homodimer composed of the fusion protein in the manufacture of a medicament; preferably, the medicament is an anti-tumor medicament; most preferably, the medicament is a medicament against B-cell lymphoma, colorectal cancer, melanoma or lung cancer.

The present disclosure also relates to a preparation method of the fusion protein, comprising steps of
(1) constructing an expression vector comprising a gene encoding the fusion protein, preferably, the expression vector is a pEE12.4 expression vector;
(2) constructing a host cell comprising the expression vector by transiently transfecting the host cell, preferably, the host cell is a 293F cell;
(3) culturing the host cell and collecting cell supernatant; and
(4) purifying the fusion protein by Protein A affinity chromatography column.

The present disclosure includes the following beneficial effects:
1. Two forms of IL-15 fusion proteins having similar structure are designed, wherein the positions of IL-15 and the sushi domain of the IL-15 receptor subunit α are exchanged at the C-terminus of Fc. The biological activities of the two IL-15 fusion proteins differ by approximately 10,000-fold.
2. A variety of linkers (the first linker) are designed to link the C-terminus of Fc to IL-15/the sushi domain of the IL-15 receptor subunit α. The basic unit of the first linker is composed of five amino acids GGGGS. The length of the first linker may directly affect the biological activity of IL-15.
3. The tumor can be completely eliminated after systemic injection of the αPD-1 Fab Fc-G₄S-IL-15-Rα fusion protein in a small MC38 tumor model (7 days after inoculation with tumor cells, the tumor size is about 30 mm³); the tumor can be substantially controlled after systemic injection of the αPD-1 Fab Fc-G₄S-IL-15-Rα fusion protein in a large MC38 tumor model (14 days after inoculation with tumor cells, the tumor size is about 100 mm³).
4. The mice treated with the fusion protein targeting drug do not show any weight loss after systemic injection of the αPD-1 Fab Fc-G₄S-IL-15-Rα fusion protein at 3 times the therapeutic dose.
5. The tumor can be substantially controlled after systemic injection of the anti-human PD-1 Fab Fc-G₄S-human IL-15-Rα or anti-human PD-1 Fab Fc-(G₄S)₃ -human IL-15-Rα fusion proteins in a A549 lung cancer tumor model of CD34+ humanized mice.

### [Term Definition]

The term "fusion protein" refers to a protein product obtained by linking the coding regions of two or more genes through genetic recombination, chemical methods or other appropriate methods and recombinantly expressing these genes under the control of the same regulatory sequence. Unless otherwise specified, the C-terminus of the first block (polypeptide) at the N-terminus of the fusion protein is linked to the N-terminus of the next block (or the linker), and so on. Therefore, the N-terminus of the block at the N-terminus of the fusion protein is the N-terminus of the fusion protein, and the C-terminus of the block at the C-terminus of the fusion protein is the C-terminus of the fusion protein.

The term "IL-15 wild type" or "wild type IL-15" refers to a naturally derived human IL-15 or a non-human mammal IL-15 or a non-mammal IL-15. It may also refer to a common IL-15 polypeptide in the art.

The term "IL-15R subunit α" may be an IL-15Rα of any species or a functional fragment thereof, such as a human IL-15Rα or a non-human mammal IL-15Rα or a non-mammal IL-15Rα. Exemplary non-human mammals include pigs, rabbits, monkeys, chimpanzees, mice, etc. Non-mammals include chickens, etc. Preferably, the IL-15R subunit α is a human IL-15Rα, and preferably an extracellular domain fragment of the human IL-15Rα.

The term "sushi domain" defines that the extracellular region of at least one sushi polypeptide-containing IL-15Rα or a fragment thereof contains an IL-15Rα sushi domain, and the extracellular region of IL-15Rα contains a domain called sushi domain (Wei et al. 2001, J. Immunol. 167:277-282). The sushi domain of IL-15Rα has a β-sheet conformation, and is encoded by exon 2 of IL-15Rα,starting from the cysteine residue (C1) encoded by the first exon 2 and ending at the cysteine residue (C4) encoded by the fourth exon 2. When considering the protein sequence of IL-15Rα in the standard N-terminus to C-terminus direction, the "sushi domain of the IL-15R subunit α" can be defined as starting from the first cysteine residue (C1) after the signal peptide and ending at the fourth cysteine residue (C4) after the signal peptide, and both residues C1 and C4 can be included in the sequence of the sushi domain.

The term "Fc" is an abbreviation for the Fc region of an immunoglobulin, and refers to the constant region of the immunoglobulin, especially the carboxy-terminus of the heavy chain constant region of the immunoglobulin, or a part thereof, which has no antigen-binding activity and is the site where the antibody interacts with effector molecules and cells. The "Fc" in the present disclosure may be any Fc or a variant thereof, derived from human or non-human mammals. For example, the Fc may comprise a combination of two or more domains of CH1, CH2, CH3 and CH4 of a heavy chain and the immunoglobulin hinge region. Fc can be derived from different species, preferably human immunoglobulins. According to the amino acid sequence of the heavy chain constant region, immunoglobulins can be divided into different classes, mainly 5 classes, including IgA, IgD, IgE, IgG and IgM. Some classes can be further divided into subclasses (isotypes), such as IgG-1, IgG-2, IgG-3 and IgG-4; or IgA-1 and IgA-2. An "Fc region" preferably comprises at least one immunoglobulin hinge region and the CH2 and CH3 domains of IgG. More preferably, it comprises a CH2 domain and a CH3 domain of IgG1, and an immunoglobulin hinge region, in which the initial amino acid position of the hinge region can be changed. In some embodiments, the Fc comprises an Fc having increased antibody-dependent cellular cytotoxicity (ADCC), antibody-dependent cellular phagocytosis (ADCP), or complement-dependent cytotoxicity (CDC) activity due to enhanced or weakened binding affinity with Fc receptors such as CD16a, CD16b, CD32a, CD16b, CD64 and C1q proteins. In other embodiments, mutations are included in the Fc domain to reduce ADCC or CDC activity. These mutations may include, but are not limited to, in the Fc domain, (1) mutations at N297, such as but not limited to N297A and N297G; (2) mutations at L234 such as L234A and L234G, and/or mutations at L235 such as L235A or L235G; (3) mutations at P329, such as P329G; or (4) mutations at D265, such as D265A; or a combination of substitutions at any or all of these positions. In other embodiments, Fc mutations (all numberings are Eu index of the Kabat numbering system) include mutation(s) that increase serum half-life. In one embodiment, the Fc has the following substitutions: T250Q, or M428L, or the T250Q/M428L double mutations in CH3 (Hinton et al., J Biol Chem. 279(8):6213-6, 2004). In other embodiments, the Fc has the M252Y/S254T/T256E triple mutations (Dall'Acqua WF et al., J Immunol 169(9):5171-80, 2002). In other embodiments, Fc has N434A mutation (Petkova SB et al., International Immunology 18(12):1759-1769, 2006.), the M428L/N434S double mutations, or the M428/N434A double mutations (Zalevsky J et al., Nat Biotechnol. 28(2):157-159, 2010). In other embodiments, the Fc region has been modified to increase its serum half-life. In some embodiments, the modification that increases serum half-life is M428L.

The term "antibody (Ab)" refers to an immunoglobulin molecule that specifically binds to a target antigen or has immunoreactivity with the target antigen, including polyclonal, monoclonal, genetically engineered and otherwise modified forms of antibodies (including but not limited to chimeric antibodies, humanized antibodies, fully human antibodies, heterologous coupled antibodies (such as bispecific, trispecific and tetraspecific antibodies, diabodies, tribodies and tetrabodies), antibody conjugates and antigen-binding fragments of antibodies (such as Fab', F(ab')2, Fab, Fv, rIgG and scFv fragments).

The term "Fab" fragment refers to one of the two identical antigen-binding fragments generated by digestion of an antibody with papain, wherein each Fab comprises a variable region of a heavy chain and a variable region of a light chain, as well as the constant region of the light chain and the first constant region of the heavy chain (CH1). As such, the term "Fab" herein refers to an antibody fragment comprising a VL region and a constant region (CL) of a light chain and a VH region and the first constant region (CH1) of the heavy chain. The Fab' fragment differs from the Fab in the addition of a few residues at the carboxy-terminus of the CH1 region of the heavy chain, comprising one or more cysteines from the antibody hinge region. Fab'-SH is a Fab' fragment in which the cysteine residue in the constant region of the heavy chain carries a free thiol group. F(ab')2 is an antibody fragment having two antigen-binding sites (two Fabs) and a part of the Fc region, produced by pepsin treatment of an intact antibody.

The term "fusion protein" refers to a protein product obtained by linking the coding regions of two or more genes through genetic recombination, chemical methods or other appropriate methods and recombinantly expressing these genes under the control of the same regulatory sequence. In the fusion protein of the present disclosure, the coding regions of two or more genes can be fused at one or several positions by sequences encoding peptide linkers or linker peptides. The term "fusion protein" of the present disclosure further includes an antibody/Fc fusion protein construct/complex, or a composition of antibody/Fc fusion protein constructs/complexes formed by a non-covalent means.

The term "the first/second linker" refers to a peptide to link IL-15 with another protein molecule or protein fragment to ensure the correct folding and stability of the protein in the present disclosure. Said another molecule includes but is not limited to IL-15Rα,Fc, Fc variant, antibody and the like.

The terms "PD1 antibody" and "αPD-1" refer to antibodies against programmed death protein 1 (PD1). Exemplary antibodies include, but are not limited to, the antibodies listed in U.S. patent Nos. 7,029,674, 7,488,802, 7,521,051, 8,008,449, 8,354,509, 8,617,546 and 8,709,417. Specific embodiments of the antibodies include BGB-A317, nivolumab (Bristol-Myers Squibb), labrolizumab (Merck), and pembrolizumab (Merck).

### BRIEF DESCRIPTION OF THE DRAWINGS

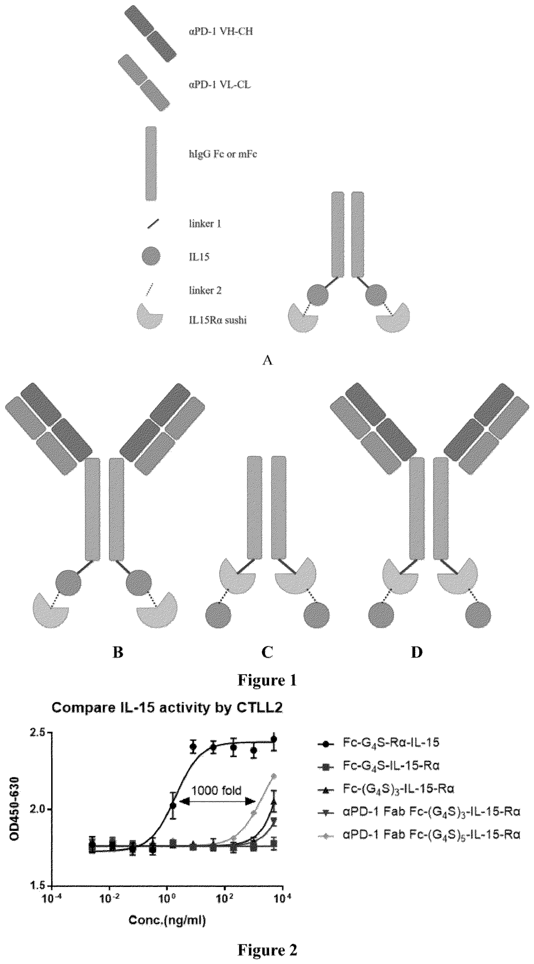

Fig. 1. A schematic diagram of the structure of the fusion protein,
   1A, A schematic diagram of the structure of Fc-G₄S-IL-15-Rα fusion protein;
   1B, A schematic diagram of the structure of αPD-1 Fab Fc-G₄S-IL-15-Rα fusion protein;
   1C, A schematic diagram of the structure of Fc-G₄S-Rα-IL-15 fusion protein;
   1D, A schematic diagram of the structure of αPD-1 Fab Fc-G₄S-Rα-IL-15 fusion protein.
Fig. 2. Comparison of biological activities of several fusion proteins with different first linkers.
Fig. 3. Comparison of the receptor-binding abilities of several fusion proteins with different first linkers.
Fig. 4. Comparison of the binding ability of Fc-G₄S-IL-15-Rα, Fc-(G₄S)3-IL-15-Rα, αPD-1 Fab Fc-(G₄S)3-IL-15-Rα and aEGFR Fab Fc-(G₄S)3-IL-15-Rα fusion proteins to CD8+ T cells in tumors.
Fig. 5. Results of treatment by intraperitoneal administration of Fc-G₄S-IL-15-Rα and Fc-G₄S-Rα-IL-15 in mice of MC38 tumor model.
Fig. 6. Changes in body weight of mice of MC38 tumor model treated by intraperitoneal administration of Fc-G₄S-IL-15-Rα and Fc-G₄S-Rα-IL-15.
Fig. 7. Survival curve of mice of MC38 tumor model treated by intraperitoneal administration of Fc-G₄S-IL-15-Rα and Fc-G₄S-Rα-IL-15.
Fig. 8. Peripheral blood lymphocyte count of mice of MC38 tumor model treated by intraperitoneal administration of Fc-G₄S-IL-15-Rα and Fc-G₄S-Rα-IL-15. The ordinate in the figure is "#/µl", which refers to the number of cells per µl of blood.
Fig. 9. Results of treatment of mice of MC38 tumor model by intraperitoneal administration of αPD-1 Fab Fc-G₄S-IL-15-Rα and Fc-G₄S-IL-15-Rα.
Fig. 10. Results of treatment of mice of MC38 tumor model by intraperitoneal administration of αPD-1 Fab Fc-G₄S-IL-15-Rα and αPD-1 Fab mix Fc-G₄S-IL-15-Rα.
Fig. 11. Results of treatment of mice of MC38 tumor model by intraperitoneal administration of αPD-1 Fab Fc-G₄S-IL-15-Rα and αPD-1 Fab Fc-G₄S-Rα-IL-15.
Figure 12. Changes in weight of mice of MC38 tumor model treated by intraperitoneal administration of αPD-1 Fab Fc-G₄S-IL-15-Rα and αPD-1 Fab Fc-G₄S-Rα-IL-15.
Fig. 13. Peripheral blood lymphocyte count of mice of MC38 tumor model treated by intraperitoneal administration of αPD-1 Fab Fc-G₄S-IL-15-Rα and αPD-1 Fab Fc-G₄S-Rα-IL-15.
Fig. 14. Results of treatment of mice of MC38 small tumor model on day 7 by intraperitoneal administration of αPD-1-IL-15 fusion proteins having different first linkers and Fc fragments.
Fig. 15. Changes in weight of mice of MC38 small tumor model on day 7 treated by intraperitoneal administration of αPD-1-IL-15 fusion proteins having different first linkers and Fc fragments.
Fig. 16. Results of treatment of mice of MC38 large tumor model on day 14 by intraperitoneal administration of αPD-1-IL-15 fusion proteins having different first linkers and Fc fragments.
Fig. 17. Results of treatment of mice of MC38 large tumor model on day 14 by intraperitoneal administration of αPD-1-IL-15 fusion proteins having different first linkers.
Fig. 18. Changes in weight of mice of MC38 large tumor model on day 14 treated by intraperitoneal administration of αPD-1-IL-15 fusion proteins having different first linkers.
Fig. 19. Peripheral blood lymphocyte count of mice of MC38 large tumor model on day 14 treated by intraperitoneal administration of αPD-1-IL-15 fusion proteins with different first linkers.
Fig. 20. Results of treatment of mice of B16 tumor model by intratumoral administration of αPD-1 Fab-Fc-(G₄S)₃-IL-15-Rα.
Fig. 21. Results of treatment of CD34+ humanized mice of A549 lung cancer tumor model by systemic administration of anti-human PD-1 Fab-Fc-G₄S-human IL-15-Rα and anti-human PD-1 Fab-Fc-(G₄S)₃-human IL-15-Rα.
Fig. 22. SDS-PAGE electrophoretogram of αPD-1 Fab-Fc-(G₄S)₃-Rα-IL-15, Fc-G₄S-Rα-IL-15, αPD-1 Fab-Fc-(G₄S)₃-IL-15-Rα and Fc-(G₄S)₃-IL-15-Rα fusion proteins.
Fig. 23. SDS-PAGE electrophoretogram of anti-human PD-1 Fab-Fc-G₄S-human IL-15-Rα and anti-human PD-1 Fab-Fc-(G₄S)₃-human IL-15-Rα fusion proteins.

### DETAILED DESCRIPTION OF THE EMBODIMENTS

### Example1. Design and construction of fusion proteins

1. Design and construction of the following eight fusion proteins:
(1) Fc-G₄S-IL-15-Rα and Fc-G₄S-Rα-IL-15 fusion proteins:
   Fc-G₄S-IL-15-IL-15Rαsushi (hereinafter referred to as Fc-G₄S-IL-15-Rα) and Fc-G₄S-IL-15Rαsushi-IL-15 (hereinafter referred to as Fc-G₄S-Rα-IL-15) have a structure as shown in Figs. 1A and 1C, wherein IL-15Rαsushi is the sushi domain of the IL-15 receptor subunit α and has an amino acid sequence as shown in Seq ID No.3 or 17, the second linker between IL-15Rα sushi and IL-15 has an amino acid sequence as shown in Seq ID No.6, and the first linker between IL-15Rαsushi or IL-15 and Fc has an amino acid sequence which is an integer multiple repeat of GGGGS, represented by (G₄S)ₙ; Fc-G₄S-IL-15-Rα has an amino acid sequence as shown in Seq ID No.8; Fc-G₄S-Rα-IL-15 has an amino acid sequence as shown in Seq ID No.7;
(2) Fc-(G₄S)₃-IL-15-Rα and Fc-(G₄S)₅-IL-15-Rα fusion proteins, obtained by further modifying the first linker of Fc-G₄S-IL-15-Rα:
   the first linker of Fc-(G₄S)3-IL-15-Rα and Fc-(G₄S)₅-IL-15-Rα is (GGGGS)₃ and (GGGGS)₅, respectively; Fc-(G₄S)₃-IL-15-Rα has an amino acid sequence as shown in Seq ID No.11; Fc-(G₄S)₅-IL-15-Rα has an amino acid sequence as shown in Seq ID No.13;
(3) αPD-1 Fab Fc-G₄S-IL-15-Rα and αPD-1 Fab Fc-G₄S-Rα-IL-15, obtained by further fusing anti-PD-1 Fab to Fc-G₄S-IL-15-Rα and Fc-G₄S-Rα-IL-15:
   αPD-1 Fab Fc-G₄S-IL-15-Rα and αPD-1 Fab Fc-G₄S-Rα-IL-15 have a structure as shown in Figs. 1B and 1D, wherein the anti-PD-1 Fab has a light chain with an amino acid sequence as shown in Seq ID No.4, 18 or 19 and a heavy chain with an amino acid sequence as shown in Seq ID No.5, 20 or 21; αPD-1Fab Fc-G₄S-IL-15-Rα has an amino acid sequence as shown in Seq ID No.10; αPD-1 Fab Fc-G₄S-Rα-IL-15 has an amino acid sequence as shown in Seq ID No.9;
(4) αPD-1 Fab Fc-(G₄S)3-IL-15-Rα and αPD-1 Fab Fc-(G₄S)₅-IL-15-Rα fusion proteins, obtained by further modifying the first linker of αPD-1 Fab Fc-G₄S-IL-15-Rα:
   αPD-1 Fab Fc-(G₄S)3-IL-15-Rα and αPD-1 Fab Fc-(G₄S)₅-IL-15-Rα have a first linker which is (GGGGS)₃ and (GGGGS)₅, respectively; αPD-1 Fab Fc-(G₄S)3-IL-15-Rα has an amino acid sequence as shown in Seq ID No.12; αPD-1 Fab Fc-(G₄S)₅-IL-15-Rα has an amino acid sequence as shown in Seq ID No.14;

### 2. Construction, transfection, expression, recovery and purification of fusion proteins

The genes of the above four forms of fusion proteins were constructed into eukaryotic expression vectors, and were transiently expressed by a single plasmid or multiple plasmids in 293F cells. The collected cell supernatant was purified by protein A. The purified protein was quantified by ELISA and Nanodrop and detected for purity by SDS-PAGE (loading 4 µg for each sample).

The specific protocols were as follows:

### 2.1. Plasmid construction

The pEE12.4-IgGκ-hIgG1 Fc plasmid comprising the mouse IgGκ signal peptide and the constant region sequence of human IgG1 was obtained from our laboratory. All the genes (IL-15, IL-15 Rα and αPD-1 Fab) involved in the present application were synthesized by a third-party commercial synthesis company, and then inserted into the pEE12.4 expression vector by enzyme digestion and ligation or homologous recombination. The plasmids were extracted with a plasmid extraction kit from TIANGEN and stored at -20°C.

### 2.2. Transient transfection for fast serum-free expression of proteins of interest

(1) 293F cells were cultured in suspension with SMM293TII medium at 37°C, 8% CO₂, and 135 rpm, and could be used for transfection when the cell density reached 4-4.5×10⁶ cells/ml, 200 ml;
(2) the cells were collected by centrifugation, washed once with Freestyle 293 medium and resuspended with 200 ml Freestyle 293 medium;
(3) 360 µg plasmids were diluted with 6 ml Freestyle 293 medium, wherein two plasmids were used to co-transfect the cells to obtain the αPD-1 fusion protein, the ratio of the plasmid expressing αPD-1 light chain to the plasmid expressing αPD-1 heavy chain was 2:1 (240 µg plasmid expressing αPD-1 light chain + 120 µg plasmid expressing αPD-1 heavy chain), and a 0.22 µm filter membrane was used for filtration and sterilization;
(4) 720 µg polyethyleneimine (PEI) was diluted with 6 ml Freestyle 293 medium, and sterilized by filtration with a 0.22 µm filter membrane;
(5) 5 ml PEI was added to the plasmids drop by drop while being vortexed, and then left for 5~10 minutes;
(6) the cell suspension was added with the plasmid/PEI mixture, and cultured in an incubator at 37°C, 8% CO₂ and 85 rpm;
(7) after 4 hours, the cell suspension was supplemented with 200 ml EX-CELLTM 293 medium and 2 mM L-Glutamine, and further cultured at a rotation speed adjusted to 135 rpm;
(8) after 24 hours, 3.8 mM valproic acid (VPA), which is cell proliferation inhibitor, was added; on day 6 after transfection, the cells were collected by centrifugation at 8000 rpm and 4°C for 2 hours, and the cell supernatant was collected for further purification.

### 2.3. Purification of proteins of interest by using Protein A

(1) Sample preparation: the collected cell supernatant was filtered with a 0.22 µm filter membrane to remove cell debris, and added with NaN₃ at a final concentration of 0.05%;
(2) the Protein A chromatographic column was rinsed and equilibrated with 10 times the column volume of double distilled water and PBS, respectively;
(3) the sample was loaded repeatedly by using a constant flow pump at a flow rate of 10 times the column volume/hour;
(4) the column was washed with 10 times the column volume of PBS to remove impurity proteins;
(5) the elution was carried out by using 0.1M Glycine (pH 2.7) to collect the eluate, and the eluted proteins were pooled and neutralized with an appropriate amount of the neutralization buffer (1M Tris, pH 9.0) (the pH value was adjusted to such an extent that, by observing the protein solution, no flocculent precipitate, which pH was about 4);
the protein was determined by SDS-PAGE electrophoresis, NanoDrop2000 and ELISA for concentration and purity, and aliquoted and stored at -80°C, and the aliquoted protein should be thawed slowly at 4°C, and cannot be frozen and thawed repeatedly.

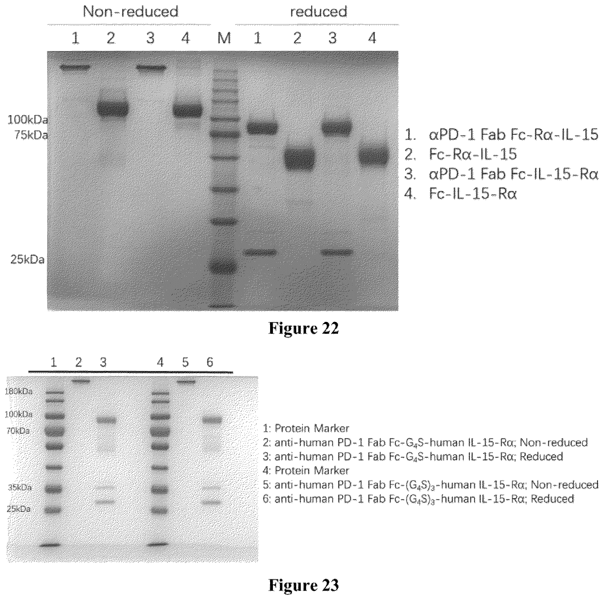

The SDS-PAGE electrophoresis results of αPD-1 Fab-Fc-(G₄S)₃-Rα-IL-15, Fc-G₄S-Rα-IL-15, αPD-1 Fab Fc-(G₄S)₃-IL-15-Rα and Fc-(G₄S)₃-IL-15-Rα fusion proteins were shown in **Fig. 22****.** The SDS-PAGE electrophoresis results of anti-human PD-1 Fab Fc-G₄S-human IL-15-Rα and anti-human PD-1 Fab Fc-(G₄S)₃-human IL-15-Rα fusion proteins were shown in **Fig. 23****.**

### Example 2. Biological function study of αPD-1-IL-15 fusion proteins

### 1. The function of promoting lymphopoiesis

The lymphocyte proliferation-promoting test (CCK8 test) included the following steps:
(1) CTLL2 cells were cultured with 1640 complete medium containing 100 U/ml commercially available recombinant IL2 cytokine;
(2) when the test was conducted, the cells were washed 2 to 3 times with complete medium without IL2, and diluted to 2×10⁴/ml;
(3) the samples of Fc-G₄S-IL-15-Rα, Fc-G₄S-Rα-IL-15, Fc-(G₄S)₃-IL-15-Rα, αPD-1Fc-(G₄S)₃-IL-15-Rα, and αPD-1 Fc-(G₄S)₅-IL-15-Rα were diluted with complete medium without IL2, with initial concentration of 5 µg/ml, 5-fold dilution, and 10 dilution gradients;
(4) 100 µl of the cell suspension and 100 µl of the samples were added into a 96-well cell culture plate, and mixed well by pipetting with a pipette tip;
(5) after cultured for 72 hours, the cells were added with 20 µl CCK8, cultured for another 3 hours, and then were detected by a microplate reader for OD values at 450 nm and 630 nm.

The detection results are shown in **Fig. 2****,** and as can been seen,
(1) the biological activity of Fc-G₄S-Rα-IL-15 was significantly increased and about 10,000 times higher than that of Fc-G₄S-IL-15-Rα, **indicating that the biological activity of the two forms of IL-15 fusion proteins are related to the relative position of IL-15 and Rα;**
(2) the biological activity of αPD-1 Fc-(G₄S)₃-IL-15-Rα was not changed compared with Fc-(G₄S)₃-IL-15-Rα, indicating that the introduction of αPD-1 antibody does not change the biological activity of IL-15 in cells with low expression of PD-1;
(3) the biological activity of αPD-1 Fc-(G₄S)₅-IL-15-Rα was higher than that of αPD-1 Fc-(G₄S)3-IL-15-Rα, **indicating that the biological activity of IL-15 in a fusion protein form is also related to the length of the first linker.**

### 2. Detection of binding ability of IL-15 fusion proteins

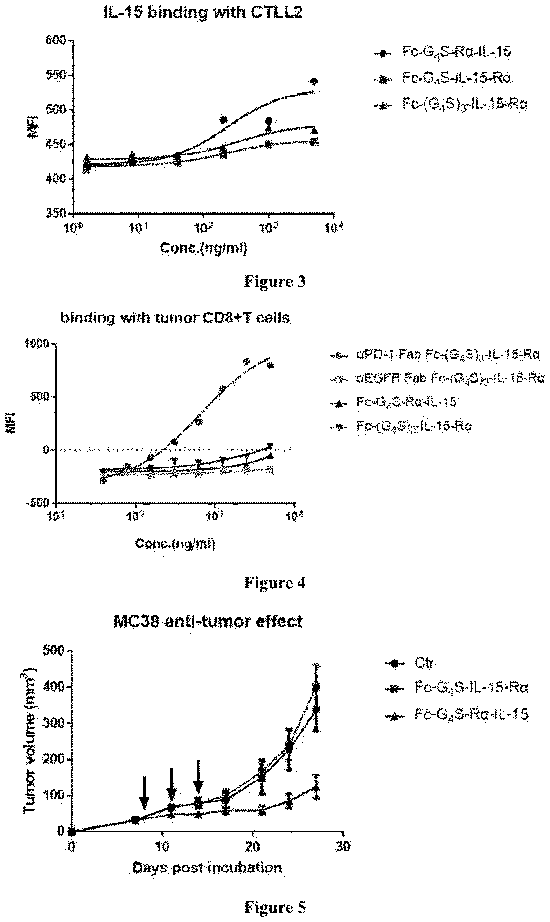

The binding ability of Fc-G₄S-IL-15-Rα, Fc-G₄S-Rα-IL-15 and Fc-(G₄S)₃-IL-15-Rα to IL-15 receptor was detected in CTLL2 reporter cell line, and the results are shown in **Fig. 3****,** indicating that the binding ability of IL-15 to its receptor is positively correlated with its biological activity, i.e., the low biological activity of Fc-G₄S-IL-15-Rα is due to the low affinity to its receptor.

The binding ability of Fc-G₄S-IL-15-Rα, Fc-(G₄S)₃-IL-15-Rα, αPD-1 Fab Fc-(G₄S)₃-IL-15-Rα and aEGFR Fab Fc-(G₄S)₃-IL-15-Rα to CD8+ T cells sorted from tumors of MC38 tumor-bearing mice was detected, and the results are shown in **Fig. 4****,** indicating that the PD-1 Fab antibody can mediate the binding of the fusion protein to CD8+ T cells.

### 3. Detection of therapeutic effect and toxic side effects of IL-15 and αPD-1-IL-15 fusion protein in a mouse tumor model

### 3.1. MC38 tumor model (administration was performed 7 days after inoculation)

### Method 1 (with a dosage of 15 µg)

5×10⁵ MC38 tumor cells were subcutaneously inoculated into the lower left side of C57 mice. When the tumor grew to 40 mm³, 15 µg Fc-G₄S-IL-15-Rα or Fc-G₄S-Rα-IL-15 was administered intraperitoneally for three times with an interval of 2 days, and PBS was given to the control group in the same way. The tumor volume was measured (volume=length × width × height/2), and the weight change and survival curve of the mice were recorded.

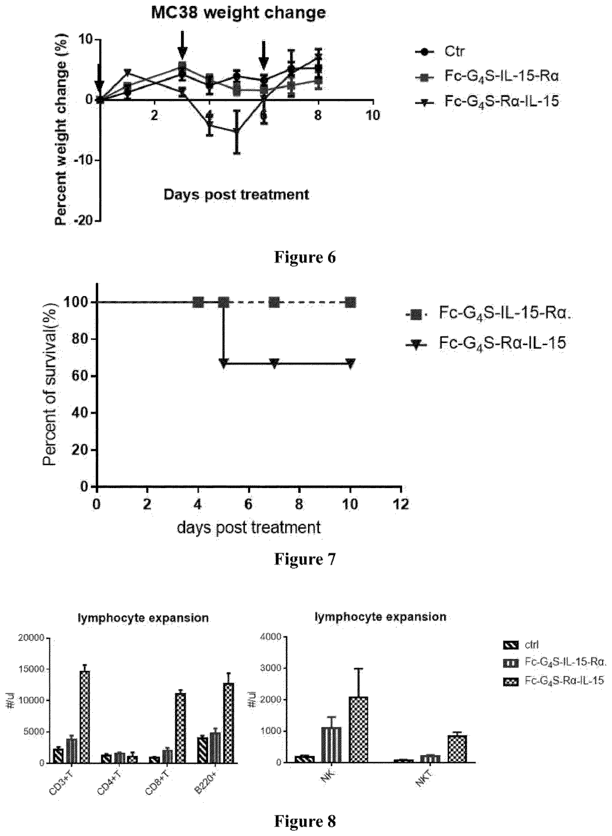

The results are shown in **Figs. 5****,** **6, 7, and 8****.**
(1) By the intraperitoneal administration of Fc-G₄S-IL-15-Rα, the tumor was not controlled, the weight was not changed, and all mice survived. It indicates that Fc-G₄S-IL-15-Rα has no significant anti-tumor effect and no toxic side effects.
(2) By the intraperitoneal administration of Fc-G₄S-Rα-IL-15, the tumor was significantly controlled, but the weight was significantly decreased, and 40% of the mice died. Moreover, in peripheral blood lymphocytes, CD8+T, B220+, NK, and NKT cells were expanded. It indicates that Fc-G₄S-Rα-IL-15 has a significant anti-tumor effect and severe toxic side effects.

### Method 2 (with a dosage of 15 µg):

5×10⁵ MC38 tumor cells were subcutaneously inoculated into the lower left side of C57 mice. When the tumor grew to 40 mm³, Fc-G₄S-IL-15-Rα (15 µg) or αPD-1 Fab Fc-G₄S-IL-15-Rα (at an equal molar mass of 30 µg) was administered intraperitoneally for 3 times with an interval of 2 days, and PBS was given to the control group in the same way. The tumor volume was measured (volume=length × width × height/2).

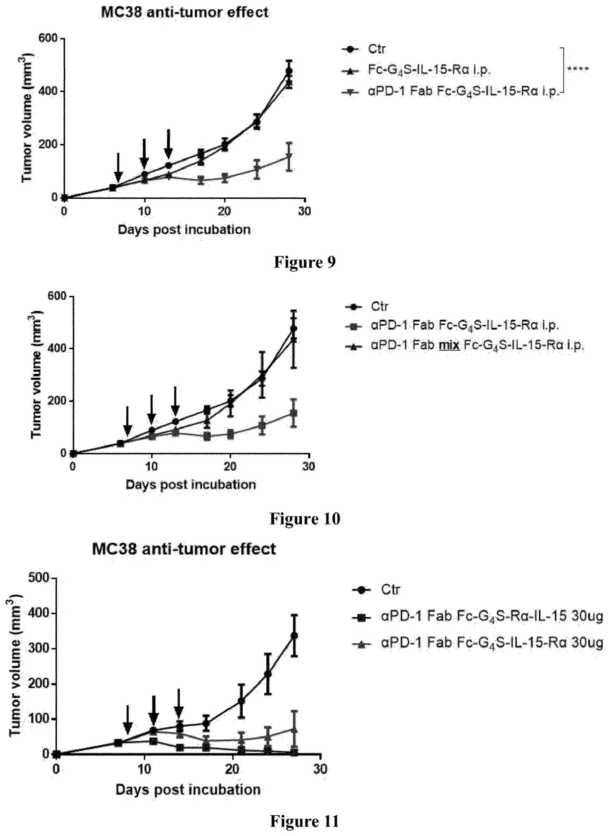

The results are shown in **Fig. 9****.** It can be seen that the tumor was not controlled in the group of intraperitoneal administration of Fc-G₄S-IL-15-Rα, but was significantly controlled in the group of intraperitoneal administration of αPD-1 Fab Fc-G₄S-IL-15-Rα. It indicates that αPD-1 Fab Fc-G₄S-IL-15-Rα can enhance the therapeutic effect through the action of αPD-1 antibody.

### Method 3 (with a dosage of 15 µg):

5×10⁵ MC38 tumor cells were subcutaneously inoculated into the lower left side of C57 mice. When the tumor grew to 40mm³, αPD-1 Fab (15 µg) + Fc-G₄S-IL-15-Rα (15µg) or αPD-1 Fab Fc-G₄S-IL-15-Rα (at an equal molar mass of 30 µg) was administered intraperitoneally for 3 times with an interval of 2 days, and PBS was given to the control group in the same way. The volume of the tumor was measured (volume=length × width × height/2).

The results are shown in **Fig. 10****.** It can be seen that the tumor was not controlled in the group of intraperitoneal administration of αPD-1 Fab+Fc-G₄S-IL-15-Rα, but was significantly controlled in the group of intraperitoneal administration of αPD-1 Fab Fc-G₄S-IL-15-Rα. It indicates that the anti-tumor effect of αPD-1 Fab Fc-G₄S-IL-15-Rα relies on the co-effect of the fusion protein of αPD-1 Fab and IL-15.

### Method 4 (with a dosage of 30 µg):

5×10⁵ MC38 tumor cells were subcutaneously inoculated into the lower left side of C57 mice. When the tumor grew to 40 mm³, αPD-1 Fab Fc-G₄S-Rα-IL-15 or αPD-1 Fab Fc-G₄S-IL-15-Rα was administered intraperitoneally for 3 times with an interval of 2 days, and PBS was given to the control group in the same way. The tumor volume was measured (volume=length × width × height/2), and the weight change of the mice was recorded.

The results are shown in **Figs. 11****,** **12, and 13****.**
(1) By the intraperitoneal administration of αPD-1 Fab Fc-G₄S-IL-15-Rα, the tumor was well controlled, the weight was not changed, and the peripheral blood lymphocytes were not significantly expanded. It indicates that αPD-1 Fab Fc-G₄S-IL-15-Rα has a significant anti-tumor effect without any toxic side effects.
(2) By the intraperitoneal administration of αPD-1 Fab Fc-G₄S-Rα-IL-15, the tumor was well controlled, but the weight loss was significant. Moreover, in peripheral blood lymphocytes, CD8+T, B220+, NK, and NKT cells were expanded. It indicates that αPD-1 Fab Fc-G₄S-Rα-IL-15 has a significant anti-tumor effect and severe toxic side effects.

### Method 5 (with a dosage of 30 µg):

5×10⁵ MC38 tumor cells were subcutaneously inoculated into the lower left side of C57 mice. When the tumor grew to 40 mm³, αPD-1 Fab Fc-G₄S-IL-15-Rα, αPD-1 Fab mFc-G₄S-IL-15-Rα, αPD-1 Fab Fc-(G₄S)₃-IL-15-Rα, αPD-1 Fab mFc-(G₄S)₃-IL-15-Rα and aEGFR Fab Fc-G₄S-IL-15-Rα were administered intraperitoneally for 3 times with an interval of 2 days, and PBS was given to the control group in the same way. The tumor volume was measured (volume=length × width × height/2), and the weight change of the mice was recorded.

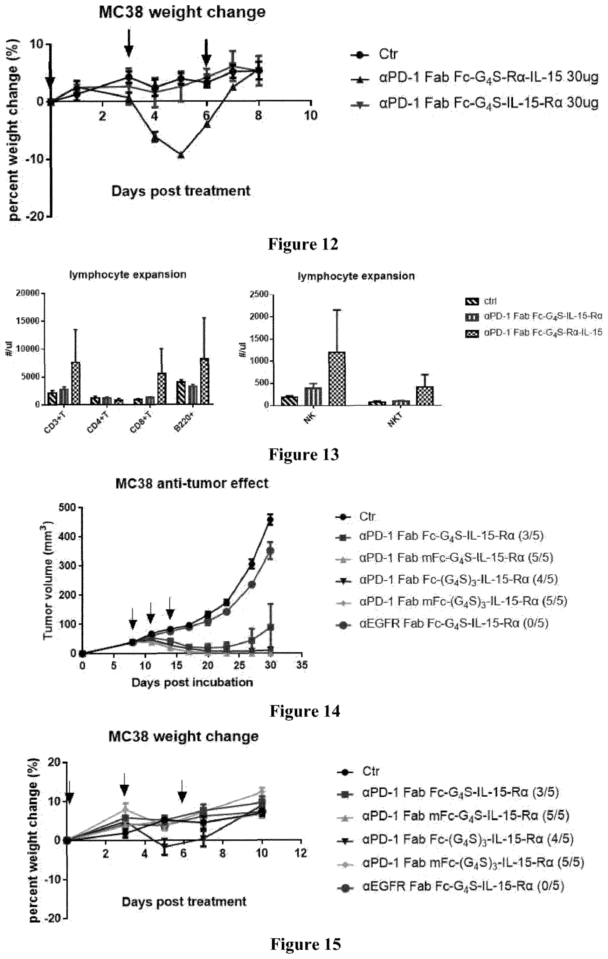

The results are shown in Figs. **14 and 15****.**
(1) By the intraperitoneal administration of αPD-1 Fab Fc-G₄S-IL-15-Rα, αPD-1 Fab mFc-G₄S-IL-15-Rα, αPD-1 Fab Fc-(G₄S)₃-IL-15-Rα, and αPD-1 Fab mFc-(G₄S)₃-IL-15-Rα, the tumors were well controlled, and the weight was not changed significantly. It indicates that αPD-1 Fab Fc-G₄S-IL-15-Rα, αPD-1 Fab mFc-G₄S-IL-15-Rα, αPD-1 Fab Fc-(G₄S)₃-IL-15-Rα, and αPD-1 Fab mFc-(G₄S)₃-IL-15-Rα have a significant anti-tumor effect without any toxic side effects.
(2) By the intraperitoneal administration of aEGFR Fab Fc-G₄S-IL-15-Rα, the tumor was not controlled and the weight was not changed. It indicates that aEGFR antibody cannot replace αPD-1 antibody to play an anti-tumor role.

### 3.2. MC38 tumor model (administration was performed 14 days after inoculation )

### Method 1 (with a dosage of 30 µg):

5×10⁵ MC38 cells were subcutaneously inoculated into the lower left side of C57 mice. When the tumor grew to 100 mm³, αPD-1 Fab Fc-G₄S-IL-15-Rα, αPD-1 Fab mFc-G₄S-IL-15-Rα, αPD-1 Fab Fc-(G₄S)₃-IL-15-Rα, αPD-1 Fab mFc-(G₄S)₃-IL-15-Rα and aEGFR Fab Fc-(G₄S)₃-IL-15-Rα were administered intraperitoneally for 3 times with an interval of 2 days, and PBS was given to the control group. The volume of the tumor was measured (volume=length × width × height/2).

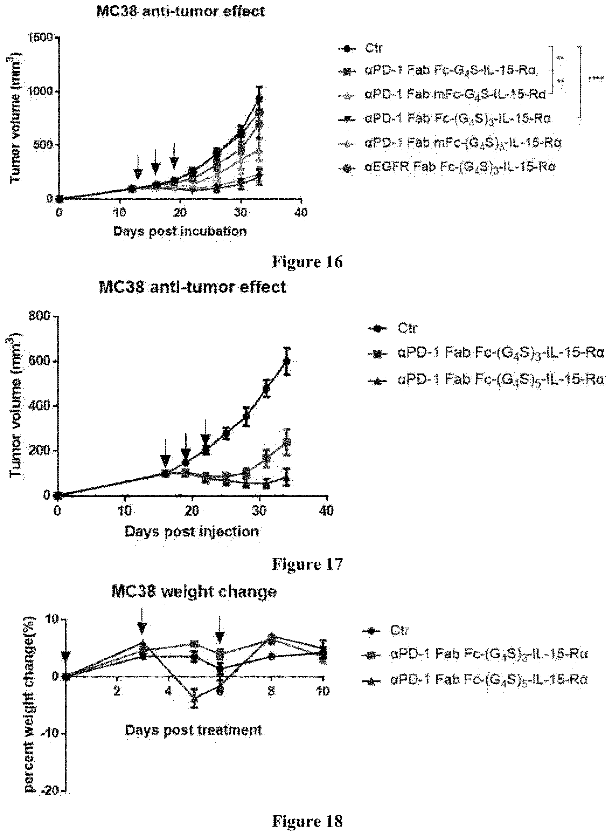

The results are shown in **Fig.16**.
(1) By the intraperitoneal administration of αPD-1 Fab Fc-(G₄S)₃-IL-15-Rα and αPD-1 Fab mFc-(G₄S)₃-IL-15-Rα, the tumors were well controlled. It indicates that when the first linker is (G₄S)₃, the fusion protein has a better anti-tumor effect.
(2) By the intraperitoneal administration of aEGFR Fab Fc-(G₄S)₃-IL-15-Rα, the tumor was not controlled and the weight was not changed. It indicates that aEGFR antibody cannot replace αPD-1 antibody to play an anti-tumor role.
(3) There was no significant difference in the anti-tumor effect between αPD-1 Fab Fc-G₄S-IL-15-Rα and αPD-1 Fab mFc-G₄S-IL-15-Rα administered intraperitoneally, indicating that mFc does not play a significant role in the anti-tumor process.

### Method 2 (with a dosage of 30 µg):

5×10⁵ MC38 cells were subcutaneously inoculated into the lower left side of C57 mice. When the tumor grew to 100 mm³, αPD-1 Fab Fc-(G₄S)₃-IL-15-Rα and αPD-1 Fab Fc-(G₄S)₅-IL-15-Rα were administered intraperitoneally for 3 times with an interval of 2 days, and PBS was given to the control group in the same way. The tumor volume was measured (volume=length × width × height/2), and the weight change of the mice was recorded.

The results are shown in **Figs. 17, 18****, and** **19****.**
(1) By the intraperitoneal administration of αPD-1 Fab Fc-(G₄S)₃-IL-15-Rα and αPD-1 Fab Fc-(G₄S)₅-IL-15-Rα, the tumors were well controlled without significant difference. It indicates that the fusion protein with the first linker of (G₄S)₅ did not have a better anti-tumor effect compared to the fusion protein with the first linker of (G₄S)₃.
(2) By the intraperitoneal administration of αPD-1 Fab Fc-(G₄S)₅-IL-15-Rα, the mice showed a slight weight loss and significant expansion of peripheral blood lymphocytes. It indicates that the fusion protein with the first linker of (G₄S)₅ has peripheral toxic side effects.

### 3.3. B16 tumor model (administration was performed 7 days after inoculation )

(1) 3×10⁵ B16 cells were subcutaneously inoculated into the lower left side of C57 mice.
(2) When the tumor grew to 30 mm³, αPD-1 Fab Fc-(G₄S)₃-IL-15-Rα was administered intratumorally for 3 times with an interval of 2 days, 30 µg per administration, and PBS was given to the control group in the same way.
(3) The volume of the tumor was measured (volume=length × width × height/2).

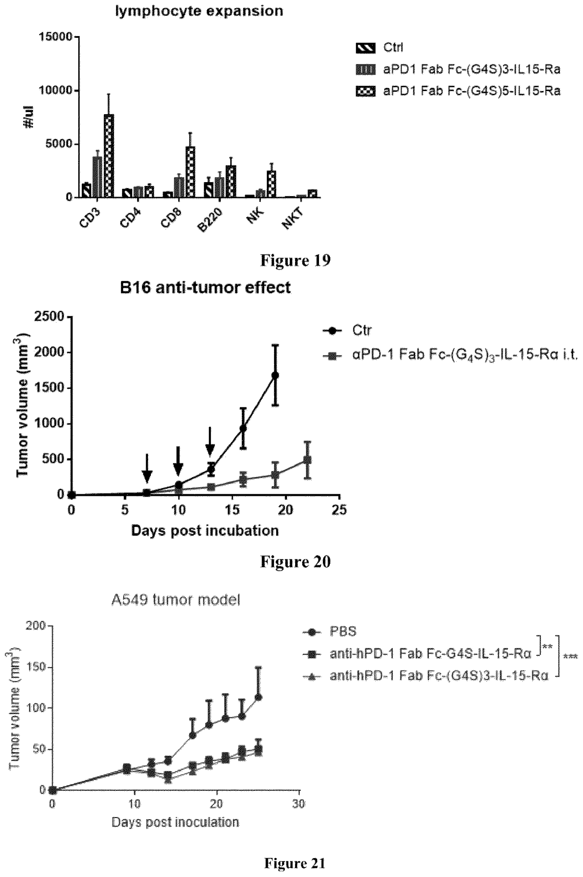

The results are shown in **Fig. 20****.**
(1) The tumor were controlled by intratumoral administration of αPD-1 Fab Fc-(G₄S)₃-IL-15-Rα. It indicates that the fusion protein has a certain anti-tumor effect on B16.

3.4. CD34+ humanized mouse A549 lung cancer model
(1) 2×10⁶ A549 cells were subcutaneously inoculated into the lower left side of CD34+ humanized mice;
(2) When the tumor grew to 30 mm³, anti-human PD-1 Fab Fc-G₄S-human IL-15-Rα or anti-human PD-1 Fab Fc-(G₄S)₃-human IL-15- Rα was systemically administered, with a dosage of 10 µg on day 10, and a dosage of 20 µg on day 17 and day 20. PBS was given to the control group in the same way.
(3) The volume of the tumor was measured (volume=length × width × height/2).

The results are shown in **Fig. 21****.**
(1) By the intraperitoneal administration of anti-human PD-1 Fab Fc-G₄S-human IL-15-Rα or anti-human PD-1 Fab Fc-(G₄S)₃-human IL-15-Rα, the tumor were controlled. It indicates that the fusion protein has an anti-tumor effect on human-derived A549 lung cancer in humanized mice.

Finally, it should be noted that the above embodiments are only used to help those skilled in the art understand the essence of the present disclosure, and are not intended to limit the protection scope of the present disclosure.

## Claims

1. A fusion protein, comprising blocks of:
(1) a first structural unit: a sushi domain of an interleukin 15 (IL-15) receptor subunit α;
(2) a second structural unit: IL-15;
(3) a third structural unit located at N-terminus of the fusion protein: an Fc fragment or a mutated Fc fragment of an antibody; and
(4) a second linker for linking the first and second structural units;
a first linker links the second and third structural units where C-terminus of the fusion protein is the first structural unit;
the first linker links the first and third structural units where the C-terminus of the fusion protein is the second structural unit;
the first linker has an amino acid sequence which is an integer multiple repeat of GGGGS, represented by (G₄S)ₙ; preferably, n is any integer from 1 to 7; more preferably, n is any integer from 1 to 5; most preferably, n is 3.

2. The fusion protein according to claim 1, wherein the fusion protein further comprises blocks of:
(5) a fourth structural unit linked to the N-terminus of the third structural unit: a Fab of a therapeutic antibody;
the therapeutic antibody includes but is not limited to: anti-PD1/PD-L1 antibody, Her2 antibody, anti-CD20 antibody, anti-CD19 antibody, anti-RANKL antibody, anti-VEGFR antibody, and anti-EGFR antibody;
preferably, the Fab of the therapeutic antibody is an anti-PD-1 Fab (a Fab of a PD1 antibody).

3. The fusion protein according to claim 2, wherein
the anti-PD-1 Fab comprises a heavy chain (variable region + constant region) and a light chain (variable region + constant region), wherein the heavy chain is located at the N-terminus of the fusion protein.

4. The fusion protein according to any one of claims 1-3, wherein
the IL-15 is a murine- or human-derived IL-15, having an amino acid sequence as shown in Seq ID No.1 or Seq ID No.15;
the sushi domain of the IL-15 receptor subunit α is a sushi domain of a murine- or human-derived IL-15 receptor subunit α; preferably, the sushi domain of the murine- or human-derived IL-15 receptor subunit α has an amino acid sequence as shown in Seq ID No.3 or Seq ID No.17;
the Fc fragment or the mutated Fc fragment has an amino acid sequence as shown in Seq ID No.2 or Seq ID No. 16;
the second linker has an amino acid sequence as shown in Seq ID No.6.

5. The fusion protein according to claim 3, wherein
the anti-PD-1 Fab is a murine- or human-derived anti-PD-1 Fab;
preferably, the anti-PD-1 Fab has a light chain with an amino acid sequence as shown in Seq ID No.4, Seq ID No.18, or Seq ID No.19;
the anti-PD-1 Fab has a heavy chain with an amino acid sequence as shown in Seq ID No.5, Seq ID No.20, or Seq ID No.21.

6. A homodimer composed of the fusion protein according to any one of claims 1-5, wherein the homodimer's monomers are linked to each other through dimerization of the third structural units.

7. The homodimer according to claim 6, wherein the homodimer is:
(1) homodimer 1 (Fc-G₄S-Rα-IL-15):
the fusion protein as the monomer, from N-terminus to C-terminus, comprises: a human-derived IgG1-Fc, a first linker (G₄S), a sushi domain of an IL-15 receptor subunit α, a second linker and IL-15; preferably, homodimer 1 has an amino acid sequence as shown in SEQ ID No.7;
(2) homodimer 2 (Fc-G₄S-IL-15-Rα):
the fusion protein as the monomer, from N-terminus to C-terminus, comprises: a human-derived IgG1-Fc, a first linker (G₄S), IL-15, a second linker, and a sushi domain of an IL-15 receptor subunit α; preferably, homodimer 2 has an amino acid sequence structure as shown in SEQ ID No.8;
(3) homodimer 3 (anti-PD-1 Fab Fc-G₄S-Rα-IL-15):
the fusion protein as the monomer, from N-terminus to C-terminus, comprises: an anti-PD-1 Fab, a human-derived IgG1-Fc, a first linker (G₄S), a sushi domain of an IL-15 receptor subunit α, a second linker, and IL-15; preferably, homodimer 3 has an amino acid sequence as shown in SEQ ID No.9;
(4) homodimer 4 (anti-PD-1 Fab Fc-G₄S-IL-15-Rα):
the fusion protein as the monomer, from N-terminus to C-terminus, comprises: an anti-PD-1 Fab, a human-derived IgG1-Fc, a first linker (G₄S), IL-15, a second linker, and a sushi domain of an IL-15 receptor subunit α, preferably, homodimer 4 has an amino acid sequence as shown in SEQ ID No.10;
(5) homodimer 5 (Fc-(G₄S)₃-IL-15-Rα):
the monomer, from N-terminus to C-terminus, comprises: a human-derived IgG1-Fc, a first linker ((G₄S)₃), a murine- or human-derived IL-15, a second linker, and a sushi domain of an IL-15 receptor subunit α; preferably, homodimer 5 has an amino acid sequence as shown in SEQ ID No.11;
(6) homodimer 6 (anti-PD-1 Fab Fc-(G₄S)₃-IL-15-Rα):
the monomer, from N-terminus to C-terminus, comprises: an anti-PD-1 Fab, a human-derived IgG1-Fc, a first linker ((G₄S)₃), IL-15, a second linker, and a sushi domain of an IL-15 receptor subunit α; preferably, homodimer 6 has an amino acid sequence as shown in SEQ ID No.12;
(7) homodimer 7 (Fc-(G₄S)₅-IL-15-Rα):
the monomer, from N-terminus to C-terminus, comprises: a human-derived IgG1-Fc, a first linker ((G₄S)₅), IL-15, a second linker, and a sushi domain of an IL-15 receptor subunit α; preferably, homodimer 7 has an amino acid sequence as shown in SEQ ID No.13;
(8) homodimer 8 (anti-PD-1 Fab Fc-(G₄S)₅-IL-15-Rα):
the monomer, from N-terminus to C-terminus, comprises: an anti-PD-1 Fab, a human-derived IgG1-Fc, a first linker ((G₄S)₅), IL-15, a second linker, and a sushi domain of an IL-15 receptor subunit α; preferably, homodimer 8 has an amino acid sequence as shown in SEQ ID No.14.

8. A nucleotide fragment encoding the fusion protein according to any one of claims 1-5.

9. Use of the fusion protein according to any one of claims 1-5 or the homodimer according to any one of claims 6-7 in the manufacture of a medicament; preferably, the medicament is an anti-tumor medicament; most preferably, the medicament is a medicament against B-cell lymphoma, colorectal cancer, melanoma or lung cancer.

10. A preparation method of the fusion protein according to any one of claims 1-5, comprising:
(1) constructing an expression vector comprising a gene encoding the fusion protein; preferably, the expression vector is a pEE12.4 expression vector;
(2) constructing a host cell comprising the expression vector by transiently transfecting the host cell; preferably, the host cell is a 293F cell;
(3) culturing the host cell and collecting cell supernatant; and
(4) purifying the fusion protein by Protein A affinity chromatography column.
